# EUROPEAN PATENT APPLICATION

(11) **EP 1 310 796 A2**
(43) Date of publication of application: **14.05.2003**
(21) Application number: 02257659.9
(22) Date of filing: 05.11.2002
(51) Int. Cl.: G01N 33/576, G01N 33/543, C07K 14/18, C07K 16/10

(54) **Reagents for the simultaneous detection of HCV core antigens and antibodies**

(30) Priority: 07.11.2001 US 347943 P; 10.10.2002 US 268562
(71) Applicant: Ortho-Clinical Diagnostics, Inc., Rochester, NY 14626-5101 (US)
(72) Inventor: Bahl, Chander, Flemington, NJ 08822 (US)
(74) Representative: Mercer, Christopher Paul

(57) **Abstract**

The present invention is directed to core protein sequences and anti-core antibodies that can be used to detect core antigen and anti core antibodies in serum collected from HCV infected individuals.

## Description

### Background of the Invention

Hepatitis C Virus (HCV) is the leading cause of transfusion-associated and community-acquired hepatitis. HCV antibodies and antigens are used in immunoassays to test blood that might be used in transfusion in order to detect the presence of HCV. This type of blood screening has significantly reduced the spread of this virus due to blood transfusion.

During the last 10 years there has been considerable progress made in improving the sensitivity of blood screening assays but there is a window period of 60-80 days from the time of infection in which there are no detectable antibodies. During this window period the persons are highly infective. To close this window, nucleic acids based tests are being developed to detect HCV infection. These nucleic acids based test are very laborious and have not yet been adapted for routine blood screening assays. Recently methods have been developed for detecting HCV core antigen. HCV core antigen can be detected in most HCV antibody negative HCV RNA positive individuals. From the published research it appears that HCV core antigen detection can be used as an earlier indication of HCV infection. (S.R. Lee et al., Vox Sanguinis, Efficacy Of A Hepatitis C Virus Core Antigen Enzyme-Linked Immunosorbent Assay For The Identification Of The 'Window-Phase' Blood Donation. 2001; 80: 19-23.) Though core antigen detection can detect HCV infection during the window period, the detection of core antigen becomes difficult once the anti-core antibodies appear. The core antigen gets complexed with the anti-core antibodies and it requires strong dissociating reagents to separate the core antigen from the antibodies.

Therefore, there remains a need for a more effective assay that detects HCV infection. We describe herein a combination assay that detects anti-HCV antibodies and HCV core antigen in a single assay will able to detect HCV infection much more effectively than the current HCV core antigen or anti-HCV antibody assays.

### Summary of the Invention

The present invention is directed to core protein sequences and anti-core antibodies that can be used to detect core antigen and anti core antibodies in serum collected from HCV infected individuals.

### Detailed Description

Anti-core antibodies are major contributors to the sensitivity of the anti-HCV antibodies assays. These antibodies are generally among the earlier antibodies to develop. HCV anti-core antibodies are directed against multiple epitopes. Most of the HCV anti-core activity can be accounted for by the amino terminal 1/3^{rd} of the HCV core protein. HCV infected individuals have antibodies to multiple epitopes in HCV core protein sequence. It is possible to detect HCV anti-core antibodies by using part of the HCV core sequence. Analysis of serum from HCV infected individuals using overlapping pentadecapeptides showed that the antibodies to HCV core are distributed over many of the peptides. Four peptides towards the amino terminus end have lot more reactivity than the peptides towards the carboxy terminus of the HCV c22-3 protein. This region of HCV core protein when further analyzed by a peptide walk analysis using overlapping octapeptides showed that 6-8 amino acids stretch of sequences between amino acids 10-43 represent multiple epitopes in HCV core sequence. Though the antibodies to HCV core were spread all across the core protein sequence, it should be possible to delete or alter amino acid sequences outside the 10-43 sequence with little impact on the ability of these modified antigens to detect anti-core antibodies. Based on the serological information a sensitive assay for the simultaneous detection of core antigen and anti-core antibodies can be designed as follows.

A mixture of HCV core protein as recombinant protein or synthetic peptides along with anti-core monoclonal or polyclonal antibodies is coated onto microwells. The anti-core antibodies selected for this assay are selected from a group of antibodies that do not recognize core sequence 10-43. This solid phase is then used to capture anti HCV core antibodies and core antigen from patient serum or plasma specimens using standard ELISA format. The captured proteins, human anti-HCV core antibodies and core protein, are detected using immunochemical methods. The anti-HCV core human antibodies are detected using peroxidase labeled anti-human IgG. The core antigen is detected using peroxidase labeled anti-core monoclonal antibodies. The anti-core antibodies used for detection are selected from a group of antibodies that do not recognize aa 10-43 and are different from the antibodies used to capture the core antigen onto the solid phase. The core antigen used to detect ant-HCV core antibodies includes most of the sequence 10-43 with or without additional HCV core sequences. The core sequences outside of aa 10-43 are modified by deleting or altering amino sequence in the region recognized by the anti-core antibodies used to capture or detect core antigen.

Therefore, we have developed an ELISA based assay for the detection of HCV infection. This new assay can detect HCV infected blood earlier than the currently used anti-HCV antibodies detection assay. The method described herein can detect HCV infection earlier because in addition to the detection of anti-HCV antibodies, this assay method also detects HCV core antigen. A specific detergent from the polyoxyethylene class is used in the HCV assay. The detergent used should be able to release the HCV core antigen from the virus by possibly disrupting the envelope protein and/or the lipid layer but this detergent should not affect the ability of the HCV recombinant antigens to capture the anti-HCV antibodies. Some of the common detergents such as N-lauryl sarcosine used in the detection of HCV core antigen destroy the ability of HCV recombinant proteins such as c22, c200, and NS5 to detect anti-HCV antibodies in a standard ELISA.

The effectiveness and advantages of the invention are further illustrated by the following examples. The examples are meant to illustrate, but not to limit, the scope and spirit of the invention.

### Example 1

In the present assay, HCV antigens c200-3, NS5 and a modified core antigen such as c22KSV 47,48 or c22KSR47L along with anti-core monoclonal antibodies (anti core murine monoclonals Pep 10, 12) were coated onto microwells in a buffer solution. After overnight incubation the buffer containing the coating proteins are removed and the microwells washed with phosphate buffered saline (PBS) containing a detergent, TWEEN 20. The antigen/antibody coated microwells were then treated with bovine serum albumin (BSA)/ sucrose solution to block the remaining protein binding sites that may be available on the microwells. After 2-24 hours, the BSA/sucrose solution is removed and the microwells air dried and stored under a descicant.

### Example 2

Two aliquotes of the specimen to be tested are diluted into 100 uL of PBS solution containing BSA, superoxide dismutase, yeast extract and 1% BRIJ 58 or BRIJ 35 or a mixture of both. The two diluted specimens were then pipetted into two HCV antigen/antibody coated wells described above. The microwells were incubated for 90 minutes at 37 degrees Celsius. The microwells were then washed five times with PBS containing 0.5% TWEEN 20 detergent. To one microwell 200uL of a murine anti-human IgG labeled with HRP was added and in the other well the same volume of anti-HCV core monoclonal antibodies (Anti Pep4) labeled with HRP was added. To each well a solution of ORTHO phenylenediamine and hydrogen peroxide was added. After incubation in the dark for 30 minutes, the reaction is stopped by adding 50 microliters of 4N sulfuric acid. An orange color in either well indicates that the specimen being tested is infected with HCV. The microwell tested with anti-human IgG tests for anti-HCV antibodies and the one tested with anti-core conjugate tests for HCV core antigen.

### Example 3

HCV core sequences are synthesized based on the HCV core sequence represented below. These sequences are prepared using recombinant techniques or standard peptide synthesis methods. 
SEQ ID NO.: 1
MSTNPKPQKKNKRNTNRRPQDVKFPGGGQIVGGVYLLPRRGPRLGVRATRKTSER
SQPRGRRQPIPKARRPEGRTWAQPGYPWPLYGNEGCGWAGWLLSPRGSRPSWGP
TDPRRRSRNLGKVIDTLTCGFADLMGYIPLVGAPLGGAARALAHGVRVLEDGVN
YATGNLPGCSFSIFLLALLSCLTVPAS (AA 1-190 HCV Core Sequence)
 
SEQ ID NO.: 2
MSTNPKPQKKNKRNTNRRPQDVKFPGGGQIVGGVYLLPRRGPR (AA1-43)
 
SEQ ID NO.: 3
KNKRNTNRRPQDVKFPGGGQIVGGVYLLPRRGPR (AA 10-43)
 
SEQ ID NO.: 1
MSTNPKPQKKNKRNTNRRPQDVKFPGGGQIVGGVYLLPRRGPRLGVRATRKTSERS
QPRGRRQPIPKARRPEGRTWAQPGYPWPLYGNEGCGWAGWLLSPRGSRPSWGPTDPR
RRSRNLGKVIDTLTCGFADLMGYIPLVGAPLGGAARALAHGVRVLEDGVNYATGNLPG
CSFSIFLLALLSCLTVPAS.
 Modifications can be made in the italicized and underlined amino acid sequence of HCV core protein. These modifications can be alterations of amino acids or deletion of sets of amino acids to remove sites of reagent anti-core antibodies. The antigen used for the assay is paired with the monoclonal antibodies that have their binding sites outside the core region 10-43. The modified core antigens along with the appropriate anti-core antibodies are used for the simultaneous detection of HCV core antigen and anti-HCV antibodies in specimens infected with HCV.

## Claims

1. An antibody/antigen composition comprising a mixture of HCV protein and modified HCV core protein and anti-core antibodies wherein said anti-core antibodies do not recognize HCV core sequence amino acids 10-43.

2. The antibody composition as claimed in claim 1 wherein said HCV protein is selected from the group consisting of: c200-3 and NS5 and said modified HCV core protein is selected from the group consisting of: c22KSV 47,48 and c22KSR47L.

3. The antibody composition as claimed in claim 1 wherein said anti-core antibodies comprise a monoclonal antibody.

4. The antibody composition as claimed in claim 1 wherein said anti-core antibodies comprise a polyclonal antibody.

5. A method of immunoassay for HCV which comprises a) immobilizing onto a solid phase an antibody/antigen composition according to claim 1, b) contacting said antibody/antigen composition with blood or serum from a patient to form an HCV antibody/antigen complex, c) adding anti-human IgG carrying a label, and d) detecting said complex.

6. The immunoassay method as claimed in claim 5 wherein said solid phase is a microwell, mircrotitre plate, membrane or bead.

7. The immunoassay method as claimed in claim 5 wherein said label is an enzyme, a dye, colored particle, radionuclide or fluorescent substance.

8. A kit containing the antibody/antigen composition as claimed in claim 1.


SEQUENCE LISTING

<110> Ortho-Clinical Diagnostics, Inc.

<120> Reagents for the simultaneous detection of HCV core antigens and antibodies

<130> CDS0287

<150> US 60/347,943

<151> 2001-11-07

<160> 3

<170> PatentIn version 3.1

<210> 1

<211> 190

<212> PRT

<213> Hepatitis C virus

<400> 1

Met Ser Thr Asn Pro Lys Pro Gln Lys Lys Asn Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala

        35                  40                  45

Thr Arg Lys Thr Ser Glu Arg Ser Gln Pro Arg Gly Arg Arg Gln Pro
    50                  55                  60

Ile Pro Lys Ala Arg Arg Pro Glu Gly Arg Thr Trp Ala Gln Pro Gly
65                  70                  75                  80

Tyr Pro Trp Pro Leu Tyr Gly Asn Glu Gly Cys Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser
            180                 185                 190

<210> 2

<211> 43

<212> PRT

<213> Hepatitis C virus

<400> 2

Met Ser Thr Asn Pro Lys Pro Gln Lys Lys Asn Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg
        35                  40

<210> 3

<211> 34

<212> PRT

<213> Hepatitis C virus

<400> 3

Lys Asn Lys Arg Asn Thr Asn Arg Arg Pro Gln Asp Val Lys Phe Pro
1               5                   10                  15

Gly Gly Gly Gln Ile Val Gly Gly Val Tyr Leu Leu Pro Arg Arg Gly
            20                  25                  30

Pro Arg